# EUROPEAN PATENT SPECIFICATION

(11) **EP 2 249 905 B1**
(45) Date of publication and mention of the grant of the patent: **19.10.2011**
(21) Application number: 09707601.2
(22) Date of filing: 06.02.2009
(51) Int. Cl.: A61M 5/315

(54) **INJECTION DEVICE HAVING MODE DEFINING ELEMENTS**
INJEKTIONSVORRICHTUNG MIT ELEMENTEN ZUM EINSTELLEN UNTERSCHIEDLICHER MODI
DISPOSITIF D'INJECTION AYANT DES ÉLÉMENTS DE DÉFINITION DE MODE

(30) Priority: 07.02.2008 EP 08151180
(43) Date of publication of application: 17.11.2010
(73) Proprietor: Novo Nordisk A/S, 2880 Bagsværd (DK)
(72) Inventor: ENGGAARD, Christian Peter, DK-3210 Vejby (DK); MARKUSSEN, Tom Hede, DK-2880 Bagsværd (DK); MICHELSEN, Freddy, DK-KGS LYNGBY 2800 (DK); SØRENSEN, Morten, DK-2880 Bagsværd (DK)
(86) International application number: PCT/EP2009/051389
(87) International publication number: WO 2009/098299

(56) References cited:
- WO-A-02/30495
- WO-A-2006/045526
- WO-A-2007/134954
- DE-A1-102004 045 326

## Description

### FIELD OF THE INVENTION

The present invention relates to an injection device for injecting predetermined doses of liquid drug, such as for injecting doses of a single fixed amount of drug, or for injecting doses of a limited number of different amounts of drug. More particularly, the present invention relates to an injection device which is driven by means of energy stored in a spring member, and in which it can easily be ensured that a correct dose is set and that the set dose is completely injected. The injection device of the present invention is particularly suitable for self-injection by the user of a liquid drug, e.g. insulin for treating diabetes.

### BACKGROUND OF THE INVENTION

WO 02/30495 discloses a pen delivery device and procedure for administering parathyroid hormone. The device allows for injection of a single predetermined dose. The procedure is a three-step method using visual indicators to set the injectable dose delivery. A dose knob is rotated from a first position to a second position. At the second position it is possible to move the dose knob axially, thereby switching the device to a state where it is possible to set a dose. This is done by rotating the dose knob to a third position corresponding to setting a predetermined dose. It is not possible to rotate the dose knob beyond the third position, thereby setting a dose which exceeds the predetermined dose. From the third position it is possible to move the dose knob axially in an opposite direction. This axial movement causes the set dose to be injected. The user has to manually push the dose knob in order to cause the set dose to be expelled. This is particularly a disadvantage for people with poor dexterity or low finger strength, such as elderly people.

### SUMMARY OF THE INVENTION

It is an object of the invention to provide an injection device which is easy to use for people with poor dexterity or low finger strength, and in which the risk of setting and/or injecting an incorrect dose is minimised.

According to the invention the above and other objects are fulfilled by providing an injection device for injecting predetermined doses of liquid drug, the injection device comprising:
- a dose setting mechanism being operable to set a predetermined dose,
- an injection mechanism being operable to cause a set dose to be injected by the injection device,
- a torsion spring being connected to the dose setting mechanism in such a manner that operation of the dose setting mechanism causes energy to be stored in the torsion spring and the torsion spring being connected to the injection mechanism in such a manner that operation of the injection mechanism causes energy which has previously been stored in the torsion spring to be released, the released energy being used for causing the set dose to be injected,
- a first mode defining element and a second mode defining element being angularly movable relative to each other in response to operation of the dose setting mechanism, and being axially movable relative to each other between a first position in which the dose setting mechanism can be operated to set a dose and a second position in which the injection mechanism can be operated to inject a set dose,
wherein the mode defining elements are shaped in such a manner that mutual axial movements between the first position and the second position are only possible at a limited number of mutual angular positions, said angular positions each corresponding to setting of a predetermined dose of liquid drug.

The injection device is adapted to inject predetermined doses of liquid drug. In the present context the term 'predetermined dose' should be interpreted to mean that only doses of a single specific amount of drug or, alternatively, a few discrete amounts of drug, can be set and injected by the injection device. This is opposed to a situation where a variable dose or a large number of discrete amounts according to the immediate requirements of the user can be set and injected. Thus, each time a dose is set by the injection device of the invention, a specific, predefined dose is set, i.e. the user can not choose which dose to set for the individual injection. However, this does not rule out that the amount of the predetermined dose can be adjusted, e.g. initially, to match the requirements of a specific user. But once the predetermined dose is set, the same dose will be set and injected each time the injection device is operated. Furthermore, the possibility of a priming function is also not ruled out.

The injection device of the invention is particularly suitable for repetitive injection, most particularly self-injection, of a liquid drug. One example of such a drug is insulin for treating diabetes. Other examples include, but are not limited to, human growth hormone (HGH) or a fluid pharmaceutical formulation comprising a glucagon-like peptide (GLP) compound used for treating type II diabetes, such as a GLP-1 compound or a GLP-2 compound.

As used herein, the term 'drug' is meant to encompass any drug-containing flowable medicine capable of being passed through a delivery means such as a hollow needle in a controlled manner, such as a liquid, solution, gel or fine suspension. Representative drugs includes pharmaceuticals such as peptides, proteins (e.g. insulin, insulin analogues and C-peptide), and hormones, biologically derived or active agents, hormonal and gene based agents, nutritional formulas and other substances in both solid (dispensed) or liquid form.

The dose setting mechanism is operable to set a predetermined dose. Thus, when the dose setting mechanism is operated the result is that the predetermined dose is set, i.e. the injection device is made ready to inject a dose of the specific, predetermined amount. The dose setting mechanism may advantageously comprise a dose knob which it may preferably be necessary to dial or rotate in order to set the dose.

The injection mechanism is operable to cause a set dose to be injected. Thus, when a predetermined dose has been set by means of the dose setting mechanism, the injection mechanism can be operated, thereby causing the set dose to be injected by the injection device. The injection mechanism may preferably comprise an injection button or similar means which can be pressed by the user in order to activate the injection mechanism. The injection mechanism may further comprise a piston rod arranged in abutment with a piston arranged in an interior part of a cartridge containing the liquid drug, in such a manner that movement of the piston rod in an axial direction causes corresponding axial movement of the piston, thereby causing an amount of liquid drug corresponding to the predetermined dose to be expelled from the cartridge, preferably via an injection needle arranged in fluid connection with the interior part of the cartridge.

A torsion spring is connected to the dose setting mechanism as well as to the injection mechanism. Thus, during setting of a dose the torsion spring is tensioned by the dose setting mechanism, and thereby energy is stored in the torsion spring. This may advantageously be done in a rotating movement, e.g. by rotating a dose knob being connected to the torsion spring. When the injection mechanism is subsequently operated in order to inject the set dose, the energy stored in the torsion spring is released. The released energy is used for causing the set dose to be injected. This may, e.g., be obtained by allowing the released energy to drive a piston rod in a distal direction. The energy may, e.g., be released by removing a blocking which is arranged to maintain the torsion spring in a tensed state until it is desired to inject the set dose, and the user therefore operates the injection mechanism.

The injection device comprises a first mode defining element and a second mode defining element. The mode defining elements are angularly movable relative to each other in response to operation of the dose setting mechanism. Thus, when the dose setting mechanism is operated to set a predetermined dose, the mode defining elements are caused to perform an angular movement relative to each other. This may advantageously be obtained by allowing one of the mode defining elements to move along with a dose knob in a rotating movement, while the other mode defining element remains rotationally fixed relative to a housing of the injection device. As an alternative, both mode defining elements may be caused to move angularly, either in opposite directions or in the same direction. It should, however, be noted that in the latter case the angle moved by the first mode defining element must differ from the angle moved by the second mode defining element in order to obtain a mutual angular movement between the mode defining elements.

The mode defining elements are further axially movable relative to each other between a first position and a second position. In the first position the dose setting mechanism can be operated to set a dose, and in the second position the injection mechanism can be operated to inject a set dose. Accordingly, the first position may be seen as defining a 'dose setting mode' and the second position may be seen as defining an 'injection mode', hence the term 'mode defining elements'.

The mode defining elements are shaped in such a manner that mutual axial movements between the first position and the second position are only possible at a limited number of mutual angular positions. Each of these angular positions corresponds to setting of a predetermined dose of liquid drug. It should be noted that one of the angular positions could advantageously correspond to 'zero dose', in which case it may be desirable to move the mode defining elements to the first position in order to be able to set a dose to be injected. This will be described further below.

Thus, the mode defining elements are not arbitrarily movable between the first position and the second position. When the mode defining elements are in the first position, i.e. during dose setting, and a user has started operating the dose setting mechanism in order to set a dose, it is not possible to move the mode defining elements into the second position unless the mutual angular position of the mode defining elements corresponds to a predetermined dose. This is because the mode defining elements are angularly movable relative to each other in response to operation of the dose setting mechanism, and therefore the mode defining elements will be moved away from a position in which they are allowed to perform mutual axial movements when the dose setting mechanism is operated. Thereby it is not possible to move the injection device into 'injection mode' until a correct dose of liquid drug has been set, and injection of an incorrect dose is therefore prevented.

Similarly, during injection of a previously set dose, it is not possible to move the mode defining elements into the first position until the mutual angular position of the mode defining elements corresponds to a predetermined dose. This predetermined dose may advantageously be 'zero dose', corresponding to the entire previously set dose having been injected. Accordingly, it can be ensured that a correct dose is not only set, but also injected.

The mode defining elements may be automatically returned to the first position when an injection has been completed. This may, e.g., be obtained by means of a spring force, e.g. originating from the torsion spring or from a separate spring member in which energy is stored during injection.

The mode defining elements may be shaped in such a manner that mutual axial movements between the first position and the second position are only possible at two mutual angular positions, one of said angular positions corresponding to setting of a predetermined non-zero dose of liquid drug, and the other of said angular positions corresponding to zero dose. According to this embodiment it is only possible to move the mode defining elements between the first position and the second position when no dose has yet been set (or a previously set dose has been completely injected) and when a predetermined fixed dose has been set.

In this case the injection device may preferably be operated in the following manner. Initially the mode defining elements are in an angular position corresponding to zero dose, and it is therefore possible to move the mode defining elements axially between the first position and the second position. When it is desired to set and inject a dose of liquid drug, the user moves the mode defining element into the first position. Thereby it is possible to operate the dose setting mechanism to set a dose. The user then starts to operate the dose setting mechanism. In doing so the mode defining elements are moved angularly relative to each other, and they are thereby moved away from the position in which axial movements are allowed. Accordingly, it is no longer possible to move the mode defining elements to the second position during dose setting. However, it may still be possible to cancel the dose, i.e. return to the zero dose position where it will be possible to move the mode defining elements back to the second position.

The dose setting mechanism is operated until the predetermined fixed dose has been set. At this point the mode defining elements are once again in a mutual angular position in which it is possible to move the mode defining elements axially between the first position and the second position. Operating the dose setting mechanism in this manner further causes energy to be stored in the torsion spring. When the user is ready to inject the dose, the mode defining elements are moved axially to the second position where it is possible to operate the injection mechanism to inject the set dose, and the injection mechanism is operated. It could be envisaged that these steps are performed simultaneously in the sense that operating the injection mechanism initially causes the mode defining elements to be moved axially to the second position, and subsequently releases the energy stored in the torsion spring. Preferably, the torsion spring is pre-biased in the sense that when the injection has been completed energy is still stored in the torsion spring, i.e. the torsion spring is still tensed. Thereby it can be ensured that sufficient energy is released during injection to cause the predetermined dose of drug to be injected completely.

Operating the injection mechanism causes the energy stored in the torsion spring during dose setting to be released. The released energy is used for driving one or more parts of the injection mechanism in such a manner that an amount of drug corresponding to the predetermined dose is injected by the injection device.

During injection the 'set dose' may be regarded as 'decreasing' from the predetermined fixed dose to 'zero dose', corresponding to the complete dose having been injected. Accordingly, when the injection is commenced the mode defining elements are moved angularly relative to each other, and they are thereby moved away from the angular position in which they can be moved axially between the first position and the second position. Thus, during injection it is not possible to move the mode defining elements axially back to the first position. Thereby it is not possible to adjust the dose during injection, and injection of a correct dose is thereby ensured.

The first mode defining element and the second mode defining element may be angularly movable relative to each other in response to operation of the injection mechanism.

The first mode defining element may be provided with a protruding part, and the second mode defining element may be provided with a limited number of angularly distributed recesses, each recess being shaped and sized to allow the protruding part of the first mode defining element to pass, thereby allowing mutual axial movements of the first mode defining element and the second mode defining element only when the angular position of the protruding part corresponds to the angular position of one of said recesses.

The mode defining elements may further be adapted to be moved to a third mutual axial position, and mutual angular movements between the mode defining elements may be prevented when the mode defining elements are positioned in the third axial position. Since mutual angular movements between the mode defining elements are prevented in the third axial position, dose setting as well as injection is also prevented when the mode defining elements are in the third position. Thus, the third axial position may be regarded as a 'pause position' which may advantageously be used during injection of a dose. In spring driven injection devices it is normally not possible for the user to pause the injection simply by stopping operation of the injection mechanism, because once the energy is released from the spring, it will cause the set dose to be injected. However, according to this embodiment of the invention the user has the possibility of temporarily stopping the injection by moving the mode defining elements axially into the third position, thereby preventing further angular movement of the mode defining elements relative to each other, and causing the injection to stop. When it is desired to continue the injection, the mode defining elements are returned to the second position, and the injection is resumed. It may be desirable to pause an injection in order to control the rate of the injection, e.g. due to pain arising from tension occurring when the liquid drug is injected subcutaneously.

As an alternative, the mode defining elements may be movable into a third mutual axial position in which it is still possible for them to perform mutual angular movements. In this case dose setting is possible when the mode defining elements are in the first position, injection is initiated when the mode defining elements are in the second position, and when the user no longer pushes the injection button the mode defining elements may be moved to the third position in which the injection is completed.

Mating sets of teeth may be formed on the first mode defining element and the second mode defining element, and said mating sets of teeth may engage when the mode defining elements are in the third axial position. In this case the mating sets of teeth prevent mutual angular movements of the first mode defining element and the second mode defining element. Alternatively, this may be obtained in another suitable way, e.g. by means of other mating elements, such as a spline connection or a key/keyway connection, or by means of a locking mechanism interlocking the first mode defining element and the second mode defining element.

The third axial position may be arranged between the first axial position and the second axial position. According to this embodiment the first position and the second position define extremes of the possible axial movement of the mode defining elements. In this case the injection device may preferably be operated in the following manner. During dose setting the mode defining elements are in the first position, and they are moved to the second position when it is desired to inject a set dose, as described above. If it is desired to pause the injection, the mode defining elements are moved back in a direction towards the first position. However, since the mode defining elements have been moved angularly away from a position in which it is possible to move them axially into the first position, this movement is stopped before the first position is reached. This stop position may advantageously be the third position. The injection mechanism may advantageously be designed in such a manner that pressing an injection button causes the mode defining elements to remain in the second position, and releasing a pressure on the injection button causes the mode defining elements to move backwards to the third position. Such a design makes it very easy for the user to operate the injection device.

According to one embodiment it may not be possible to operate the dose setting mechanism to set a dose when the mode defining elements are in the third axial position. Thereby it is prevented that the dose is adjusted during a pause in the injection, and the risk of injecting an incorrect dose is reduced even further.

Movement of the mode defining elements from the third axial position to the first axial position may only be possible when a previously set dose has been completely injected. As described above, it is thereby prevented that the mode defining elements can be moved into a position where it is possible to adjust the dose during a pause in the injection, and it can thereby be ensured that the predetermined dose which has been set is also the dose which is subsequently injected.

The mode defining elements may be arranged to return automatically to the first position upon completion of an injection. This may advantageously be obtained by means of mechanical biasing means, such as a spring member arranged to bias the mode defining elements towards the first position. The spring member may, e.g., be the torsion spring. Alternatively, it may be a separate spring member, e.g. arranged in such a manner that energy is stored in the separate spring member during injection of a dose, the stored energy being released when the injection has been completed and the mode defining elements are once again allowed to move into the first position.

In a specific embodiment of the invention the user is able to pre-define the size of the predetermined dose, i.e. to calibrate the injection device such that a specific selected dose out of a small number, e.g. two to five, of different doses is being set each time the dose setting mechanism is operated. In this case, the injection device is provided with adjustment means, e.g. in the form of a rotatable adjustment ring, capable of being manipulated in such a manner that the user, or his/her doctor, can easily change from one predetermined dose size to another.

The adjustment means may comprise a position defining geometry adapted to be positioned such as to define the mutual angular positions of the first and the second mode defining elements at which the first and the second mode defining elements are able to perform relative axial movements. This may be realised by variably limiting the possible mutual angular movements of the first and the second mode defining elements. The adjustment means may constitute a part of or the entire second mode defining element in which case the position defining geometry may limit the angular movement of the first mode defining element relative to the adjustment means. In case the adjustment means constitutes a part of the second mode defining element the position defining geometry may be adapted to define the mutual angular positions of the first and the second mode defining elements at which the first and the second mode defining elements are able to perform relative axial movements only when taking up certain positions relative to another part of the second mode defining element.

### BRIEF DESCRIPTION OF THE DRAWINGS

The invention will now be described in further detail with reference to the accompanying drawings in which
Fig. 1 is a perspective view of an injection device according to an embodiment of the invention,
Fig. 2 is a perspective view of selected parts of the injection device of Fig. 1,
Fig. 3 is a cross sectional view of the injection device of Fig. 1,
Figs. 4-7 show mode defining elements of the injection device of Fig. 1 in various positions during operation of the injection device,
Fig. 8 is a perspective view of an injection device according to another embodiment of the invention having adjustment means for pre-defining the size of the predetermined dose,
Figs. 9a and 9b show different views of an adjustment ring of the injection device of Fig. 8,
Fig. 10 is a perspective view of the proximal end of the injection device of Fig. 8 with certain components removed, and
Fig. 11 is a perspective view of the first mode defining element of the injection device of Fig. 8.

### DESCRIPTION OF EXEMPLARY EMBODIMENTS

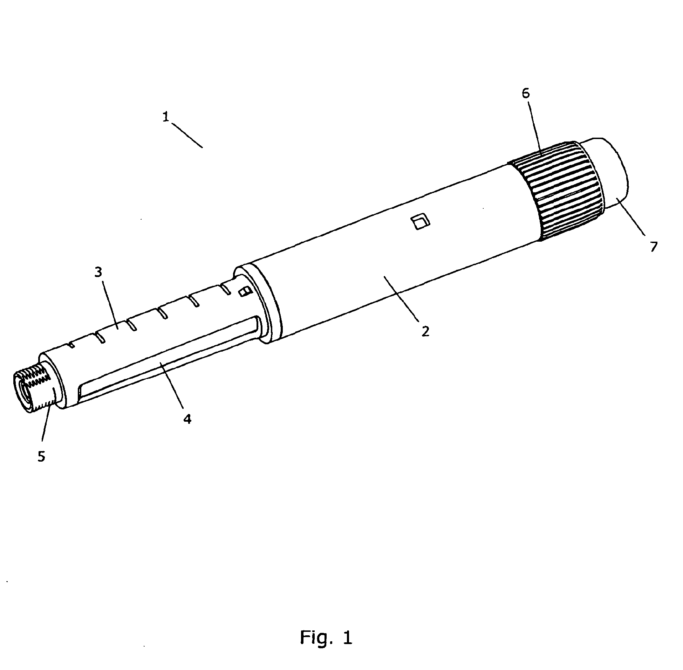

Fig. 1 is a perspective view of an injection device 1 according to an embodiment of the invention. The injection device 1 comprises a housing 2 and a cartridge holding part 3 holding a cartridge 4 containing liquid drug. At a distal end of the cartridge holding part 3 a threaded portion 5 is arranged which can be used for attaching an injection needle to the injection device 1. The injection device 1 further comprises a dose knob 6 and an injection button 7, both arranged at a proximal end of the injection device 1. In order to set a dose, the dose knob 6 must be rotated, and in order to inject a set dose, the injection button 7 must be pressed in a distal direction, i.e. towards the housing 2.

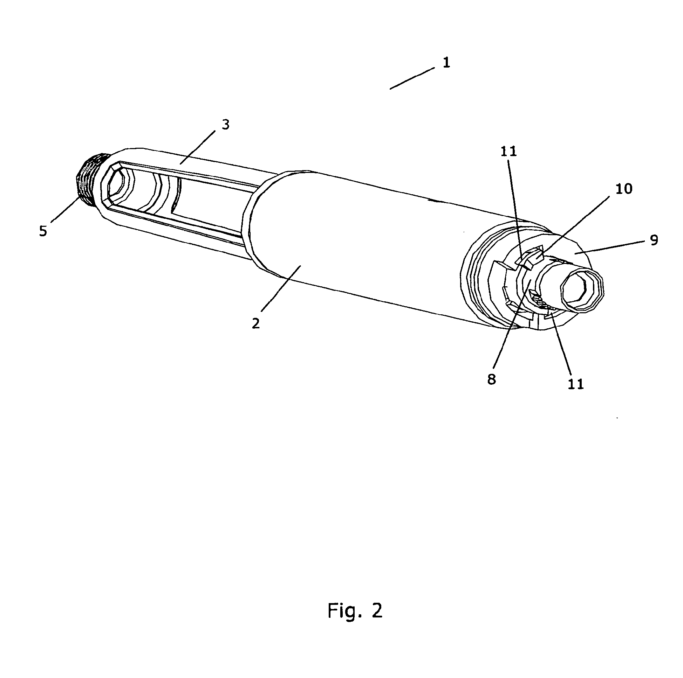

Fig. 2 is a perspective view of selected parts of the injection device 1 of Fig. 1. More particularly, the dose knob and the injection button have been removed in order to allow the parts arranged underneath the dose knob and the injection button to be seen.

In Fig. 2 it can be seen that the injection device 1 comprises a first mode defining element 8 and a second mode defining element 9. The first mode defining element 8 is provided with a protruding part 10, and the second mode defining element 9 is provided with two recesses 11. In Fig. 2 the protruding part 10 is arranged at an angular position corresponding to the angular position of one of the recesses 11. The mode defining elements 8, 9 are rotationally movable relative to each other. Accordingly, the mode defining elements 8, 9 may be moved into or away from a mutual angular position where the protruding part 10 and one of the recesses 11 are arranged at corresponding angular positions. At such positions the mode defining elements 8, 9 are allowed to move axially relative to each other, thereby positioning the protruding part 10 on either a proximal or a distal side of the second mode defining element 9.

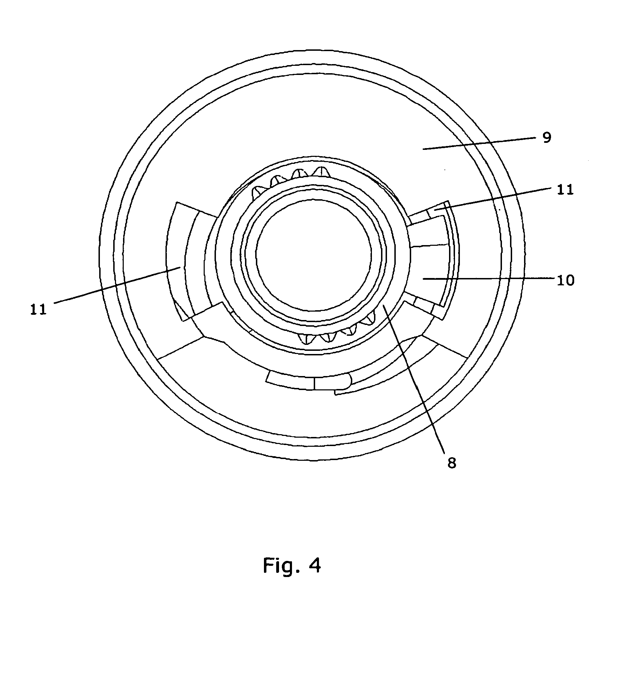

When the protruding part 10 is arranged on a proximal side of the second mode defining element 9 it is possible to set a dose, i.e. the mode defining elements 8, 9 are in a first position. When the protruding part 10 is arranged on a distal side of the second mode defining element 9 it is possible to inject a previously set dose, i.e. the mode defining elements 8, 9 are in a second position. The operation of the injection device 1 will be described in further detail below with reference to Figs. 4-7.

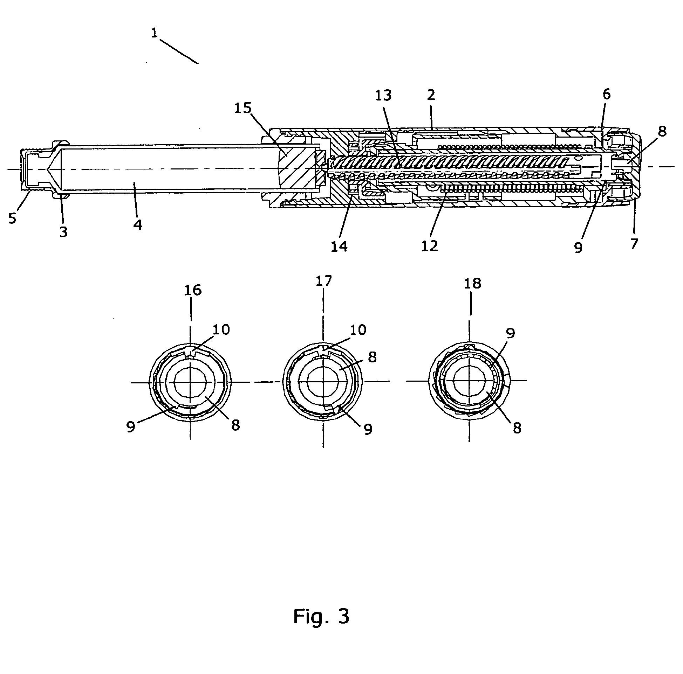

Fig. 3 is a cross sectional view of the injection device 1 of Fig. 1. When it is desired to set a dose the injection button 7 is initially pulled in a proximal direction, i.e. out of the housing 2. Thereby the mode defining elements 8, 9 are moved axially relative to each other into the first position as described above. Then the dose knob 6 is rotated, thereby causing mutual rotational movement of the first mode defining element 8 and the second mode defining element 9. Furthermore, this rotational movement of the dose knob 6 causes energy to be stored in torsion spring 12. When the predetermined dose has been set in this manner, the mode defining elements 8, 9 are once again in a mutual angular position which allows mutual axial movements of the mode defining elements 8, 9.

When the user is ready to inject the set dose, he or she pushes the injection button 7 in a distal direction, i.e. towards the housing 2. This causes the mode defining elements 8, 9 to be moved axially relative to each other into the second position. Furthermore, this movement of the injection button 7 causes the energy which was stored in the torsion spring 12 during dose setting to be released. The released energy rotates piston rod 13 which is threadedly connected to nut 14. Accordingly, the rotational movement of the piston rod 13 has the consequence that piston rod 13 is moved in the thread of the nut 14, thereby performing an axial movement in a distal direction. Thereby the piston rod 13 pushes piston 15 in a distal direction, and liquid drug is expelled from the cartridge 4.

If the user wishes to pause the injection, he or she can simply relieve the pressure on the injection button 7. This will cause the first mode defining element 8 to move in a proximal direction. However, since the mode defining elements 8, 9 are not in a mutual angular position which allows mutual axial movements between the first position and the second position, the first mode defining element 8 is prevented from moving as far as the first position. Instead, engaging sets of teeth (not visible in Fig. 3) formed on the first mode defining element 8 and the second mode defining element 9 engage, thereby preventing further mutual rotational movements of the mode defining elements 8, 9. Thereby the injection is stopped, but it can be resumed by once again pressing the injection button 7 in a distal direction. This pause function will be described further below with reference to Figs. 4-7.

Fig. 3 further shows cross sectional views of the mode defining elements 8, 9 in three different mutual positions, corresponding to three different steps of the dose setting and injection procedure. The first cross sectional view 16 shows the mode defining elements 8, 9 in a position where the injection device 1 is ready for setting a dose, but dose setting has not yet been commenced. It can be seen that the protruding part 10 of the first mode defining element 8 is positioned in such a manner that it is possible to move the mode defining elements 8, 9 axially relative to each other.

The second cross sectional view 17 shows the mode defining elements 8, 9 during dose setting. In this position it is not possible to move the mode defining elements 8, 9 axially relative to each other.

The third cross sectional view 18 shows the mode defining elements 8, 9 during injection of a dose. It can be seen that the protruding part is positioned at the opposite side of the second mode defining element 9, since it is not visible, i.e. the mode defining elements 8, 9 are in the second position.

Fig. 4 is an end view of the injection device of Fig. 1. The first mode defining element 8 and the second mode defining element 9 can be seen. The second mode defining element 9 is provided with two recesses 11 arranged angularly with approximately 180° between them. The protruding part 10 of the first mode defining element 8 is arranged at an angular position corresponding to the angular position of one of the recesses 11, thereby allowing mutual axial movements of the mode defining elements 8, 9. In Fig. 4 a predetermined dose has been set, and the injection device is ready for injecting the set dose.

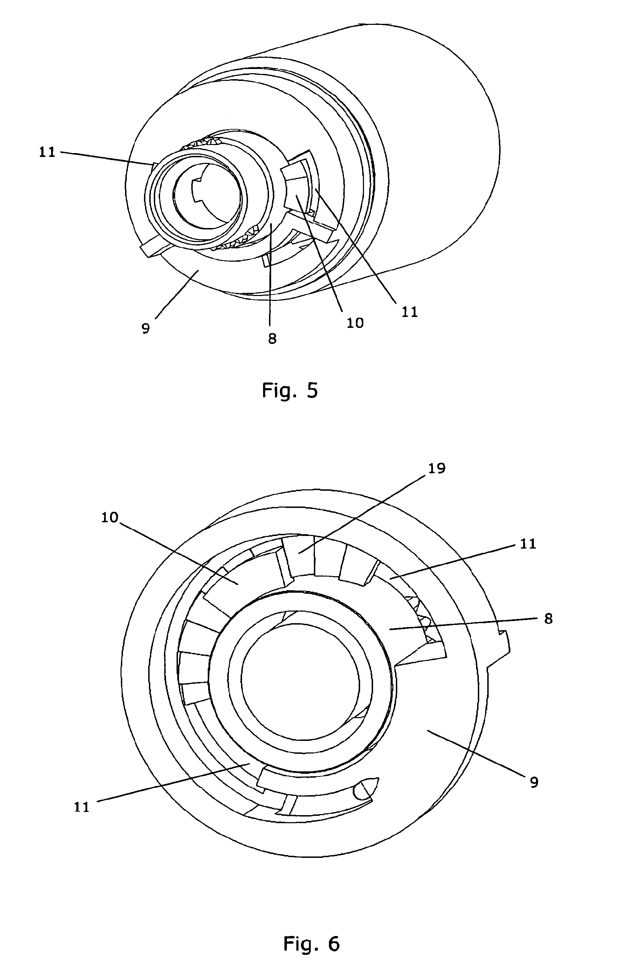

Fig. 5 is a perspective view showing the mode defining elements 8, 9 in the same mutual position as in Fig. 4.

Fig. 6 is a perspective view showing the mode defining elements 8, 9 from an opposite direction and during injection of a dose. It can be seen that the protruding part 10 of the first mode defining element 8 is arranged angularly at a position which does not correspond to the angular position of one of the recesses 11 of the second mode defining element 9. Accordingly, it is not possible to move the mode defining elements 8, 9 axially relative to each other in such a manner that the protruding part 10 is moved to the opposite side of the second mode defining element 9, i.e. into the first position. A set of teeth 19 is formed on the second mode defining element 9. In Fig. 6 the protruding part 10 is arranged in engagement with the teeth 19. Thereby mutual angular movements between the first mode defining element 8 and the second mode defining element 9 are prevented. Accordingly, the injection which is in progress has been temporarily stopped. Since it is not possible to move the mode defining elements 8, 9 axially into the first position, it is not possible to adjust the set dose during such a pause, and the only way to move the mode defining elements 8, 9 out of this position is to once again press the injection button to move the protruding part 10 out of engagement with the teeth 19. This will once again allow the mode defining elements 8, 9 to perform mutual angular movements, and the injection can be completed.

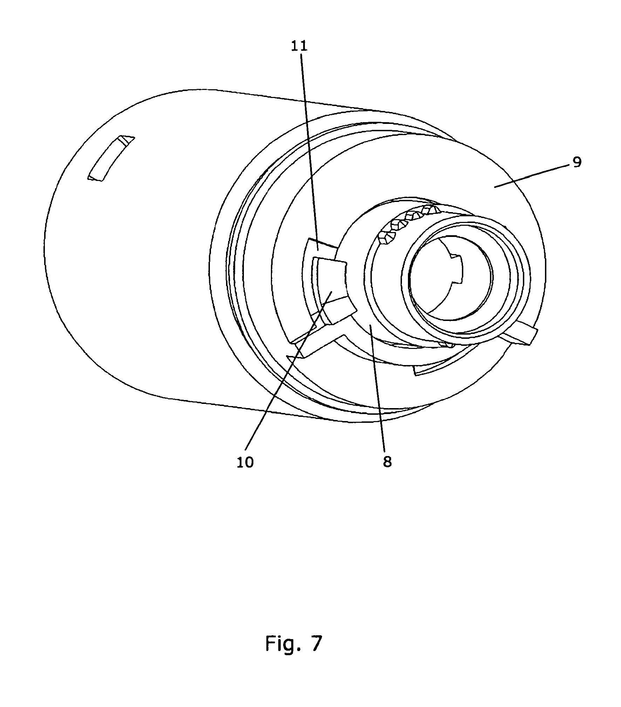

Fig. 7 is a perspective view of the mode defining elements 8, 9 in a position where the injection has been completed. Once again the protruding part 10 of the first mode defining element 8 is arranged angularly at a position corresponding to the angular position of one of the recesses 11 of the second mode defining element 9, thereby allowing mutual axial movements of the mode defining elements 8, 9.

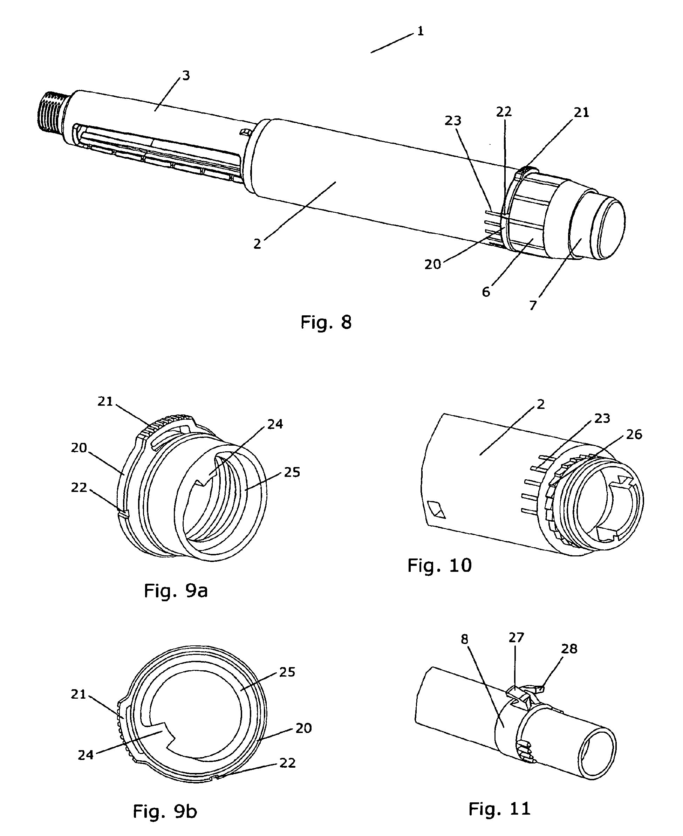

Fig. 8 is a perspective view of an embodiment of the invention in which the injection device 1 is provided with an adjustment ring 20 for pre-adjusting the size of the predetermined dose being set when the dose knob 6 is rotated to set a dose. Adjustment ring 20 has a grip interface 21 for easy manipulation by the user and an indication recess 22 the position of which relative to the housing 2 indicates the chosen dose size. Markings 23 are provided on the housing 2 to denote the possible positions of the indication recess 22 corresponding to the various predetermined dose sizes offered by the injection device 1.

Figs. 9a and 9b show a perspective view, respectively a proximal end view, of the adjustment ring 20. A boss 24 and a circumferential plateau 25 are provided for guiding the movements of the first mode defining element 8 during dose setting.

Fig. 10 is a perspective view of the proximal end of the housing 2 where the adjustment ring 20, the dose knob 6 and the injection button 7 have been removed. A set of teeth 26 extending circumferentially just proximal of the markings 23 are adapted to engage with one or more mating structures (not visible) on the adjustment ring 20 to guide the positioning of the indication recess 22 when the adjustment ring 20 is rotated to define the size of the predetermined dose.

Fig. 11 is a perspective view of the first mode defining element 8 according to this particular embodiment. The first mode defining element 8 is provided with oppositely directed snap arms 27, 28.

A rotation of the adjustment ring 20 with respect to the housing 2, e.g. in order to position the indication recess 22 at the specific marking 23 representing the desired predetermined dose size, will cause the boss 24 to rotate accordingly. When the indication recess 22 is positioned at the desired marking 23 the boss 24 takes up a certain angular position relative to the housing 2 and the first mode defining element 8, thereby defining the extent to which the first mode defining element 8 is able to undergo relative angular movement with respect to the adjustment ring 20 during dose setting.

Having thus defined the size of the predetermined dose when the user subsequently rotates the dose knob 6 to set the predetermined dose the snap arms 27, 28 ride along the circumferential plateau 25, and the first mode defining element 8 is prevented from moving axially relative to the adjustment ring 20 due to the snap arm 27 engaging with the surface of the circumferential plateau 25, until the side of the snap arm 28 abuts with the boss 24 and prevents further rotation of the first mode defining element 8. At that point the predetermined dose is set and, as seen from Fig. 9b, the circumferential plateau 25 has tapered off as a consequence of an increased portion of the adjustment ring 20 adjacent the boss 24. The increased portion of the adjustment ring 20 forces the snap arm 27 to deflect radially inwards, thereby enabling relative axial movement between the first mode defining element 8 and the adjustment ring 20. Pushing the injection button 7 towards the housing 2 moves the first mode defining element 8 axially with respect to the adjustment ring 20 and an injection is performed as described above. During injection, the first mode defining element 8 is prevented from moving axially with respect to the adjustment ring 20 due to the snap arm 28 engaging with a circumferentially extending movement restricting structure (not shown) distally of the circumferential plateau 25. The circumferentially extending movement restricting structure is configured like the circumferential plateau 25 such that it tapers off at a certain point. At this point the circumferentially extending movement restricting structure no longer engages with the snap arm 28 whereby the first mode defining element 8 is enabled to move axially relative to the adjustment ring 20. Notably, this point corresponds to the end-of-dose state of the injection device 1 which means that only when the entire predetermined dose has been injected is the first mode defining element 8 again allowed to move axially relative to the adjustment ring 20. At that point the first mode defining element 8 can be moved back to a position proximally of the adjustment ring 20 and a next dose can be set.

## Claims

1. An injection device for injecting predetermined doses of liquid drug, the injection device comprising:
- a dose setting mechanism being operable to set a predetermined dose,
- an injection mechanism being operable to cause a set dose to be injected by the injection device,
- a torsion spring being connected to the dose setting mechanism in such a manner that operation of the dose setting mechanism causes energy to be stored in the torsion spring and the torsion spring being connected to the injection mechanism in such a manner that operation of the injection mechanism causes energy which has previously been stored in the torsion spring to be released, the released energy being used for causing the set dose to be injected,
- a first mode defining element and a second mode defining element being angularly movable relative to each other in response to operation of the dose setting mechanism, and being axially movable relative to each other between a first position in which the dose setting mechanism can be operated to set a dose and a second position in which the injection mechanism can be operated to inject a set dose,
wherein the mode defining elements are shaped in such a manner that mutual axial movements between the first position and the second position are only possible at a limited number of mutual angular positions, said angular positions each corresponding to setting of a predetermined dose of liquid drug.

2. An injection device according to claim 1, wherein the mode defining elements are shaped in such a manner that mutual axial movements between the first position and the second position are only possible at two mutual angular positions, one of said angular positions corresponding to setting of a predetermined non-zero dose of liquid drug, and the other of said angular positions corresponding to zero dose.

3. An injection device according to claim 1 or 2, wherein the first mode defining element is provided with a protruding part, and the second mode defining element is provided with a limited number of angularly distributed recesses, each recess being shaped and sized to allow the protruding part of the first mode defining element to pass, thereby allowing mutual axial movements of the first mode defining element and the second mode defining element only when the angular position of the protruding part corresponds to the angular position of one of said recesses.

4. An injection device according to any of the preceding claims, wherein the mode defining elements are further adapted to be moved to a third mutual axial position, and wherein mutual angular movements between the mode defining elements is prevented when the mode defining elements are positioned in the third axial position.

5. An injection device according to claim 4, wherein mating sets of teeth are formed on the first mode defining element and the second mode defining element, and wherein said mating sets of teeth engage when the mode defining elements are in the third axial position.

6. An injection device according to claim 4 or 5, wherein the third axial position is arranged between the first axial position and the second axial position.

7. An injection device according to any of claims 4-6, wherein it is not possible to operate the dose setting mechanism to set a dose when the mode defining elements are in the third axial position.

8. An injection device according to any of claims 4-7, wherein movement of the mode defining elements from the third axial position to the first axial position is only possible when a previously set dose has been completely injected.

9. An injection device according to any of the preceding claims, wherein the mode defining elements are arranged to return automatically to the first position upon completion of an injection.

10. An injection device according to claim 9, wherein the automatic return to the first position is obtained by means of mechanical biasing means.

11. An injection device according to any of the preceding claims, further comprising adjustment means for pre-adjusting the size of the predetermined dose.

12. An injection device according to claim 11, wherein the adjustment means comprises an adjustable position defining geometry adapted to define a limit of relative angular movement between the first mode defining element and the second mode defining element during dose setting.

13. An injection device according to claim 12, wherein the adjustment means constitutes at least a part of the second mode defining element.

## Patentansprüche

1. Injektionsvorrichtung zum Injizieren von vorbestimmten Dosen an flüssigem Arzneistoff, wobei die Injektionsvorrichtung umfasst:
- einen Dosiseinstellmechanismus, der zum Einstellen einer vorbestimmten Dosis betriebsfähig ist,
- einen Injektionsmechanismus, der zum Bewirken dessen betriebsfähig ist, dass eine eingestellte Dosis durch die Injektionsvorrichtung injiziert wird,
- eine Drehfeder, die mit dem Dosiseinstellmechanismus in einer derartigen Weise verbunden ist, dass der Betrieb des Dosiseinstellmechanismus bewirkt, dass in der Drehfeder Energie gespeichert wird, und die Drehfeder in einer derartigen Weise mit dem Injektionsmechanismus verbunden ist, dass der Betrieb des Injektionsmechanismus bewirkt, dass im Vorhinein in der Drehfeder gespeicherte Energie freigesetzt wird, wobei die freigesetzte Energie zum Bewirken dessen verwendet wird, dass die eingestellte Dosis injiziert wird,
- ein erstes modusdefinierendes Element und ein zweites modusdefinierendes Element, die als Antwort auf den Betrieb des Dosiseinstellmechanismus in Bezug zu einander winkelförmig beweglich sind und zwischen einer ersten Position, in welcher der Dosiseinstellmechanismus zum Einstellen einer Dosis betrieben werden kann, und einer zweiten Position, in welcher der Injektionsmechanismus zum Injizieren einer eingestellten Dosis betrieben werden kann, zueinander axial beweglich sind,
wobei die modusdefinierenden Elemente in einer derartigen Weise gestaltet sind, dass gegenseitige axiale Bewegungen zwischen der ersten Position und der zweiten Position nur mit einer begrenzten Anzahl an gegenseitigen Winkelpositionen möglich sind, wobei die Winkelpositionen jeweils der Einstellung einer vorbestimmten Dosis an flüssigem Arzneistoff entsprechen.

2. Injektionsvorrichtung nach Anspruch 1, wobei die modusdefinierenden Elemente in einer derartigen Weise gestaltet sind, dass gegenseitige axiale Bewegungen zwischen der ersten Position und der zweiten Position nur mit zwei gegenseitigen Winkelbewegungen möglich sind, wobei eine der Winkelpositionen der Einstellung einer vorbestimmten Dosis an flüssigem Arzneistoff ungleich Null und die andere der Winkelpositionen einer Dosis gleich Null entspricht.

3. Injektionsvorrichtung nach Anspruch 1 oder 2, wobei das erste modusdefinierende Element mit einem hervorstehenden Teil und das zweite modusdefinierende Element mit einer beschränkten Anzahl an winkelförmig verteilten Vertiefungen versehen ist, wobei jede Vertiefung derart gestaltet und bemessen ist, dass dem hervorstehenden Teil des ersten modusdefinierenden Elements die Passage ermöglicht wird, wodurch gegenseitige axiale Bewegungen des ersten modusdefinierenden Elements und des zweiten modusdefinierenden Elements nur ermöglicht werden, wenn die Winkelposition des hervorstehenden Teils der Winkelposition von einer der Vertiefungen entspricht.

4. Injektionsvorrichtung nach einem der vorangehenden Ansprüche, wobei die modusdefinierenden Elemente des Weiteren derart angepasst sind, dass sie zu einer dritten gegenseitigen axialen Position bewegt werden, und wobei die gegenseitigen winkelförmigen Bewegungen zwischen den modusdefinierenden Elementen verhindert wird, wenn sich die modusdefinierenden Elemente in der dritten axialen Position befinden.

5. Injektionsvorrichtung nach Anspruch 4, wobei auf dem ersten modusdefinierenden Element und dem zweiten modusdefinierenden Element zueinander passende Zahnsätze ausgebildet sind, und wobei die zueinander passenden Zahnsätze eingreifen, wenn sich die modusdefinierenden Elemente in der dritten axialen Position befinden.

6. Injektionsvorrichtung nach Anspruch 4 oder 5, wobei die dritte axiale Position zwischen der ersten axialen Position und der zweiten axialen Position angeordnet ist.

7. Injektionsvorrichtung nach einem der Ansprüche 4-6, wobei der Betrieb des Dosiseinstellmechanismus zum Einstellen einer Dosis nicht möglich ist, wenn sich die modusdefinierenden Elemente in der dritten axialen Position befinden.

8. Injektionsvorrichtung nach einem der Ansprüche 4-7, wobei eine Bewegung der modusdefinierenden Elemente von der dritten axialen Position zu der ersten axialen Position nur möglich ist, wenn eine im Vorhinein eingestellte Dosis vollständig injiziert worden ist.

9. Injektionsvorrichtung nach einem der vorangehenden Ansprüche, wobei die modusdefinierenden Elemente derart angeordnet sind, dass sie nach vollständiger Injektion automatisch zu der ersten Position zurückkehren.

10. Injektionsvorrichtung nach Anspruch 9, wobei die automatische Rückkehr zu der ersten Position mithilfe eines mechanischen Vorspannmittels erhalten wird.

11. Injektionsvorrichtung nach einem der vorangehenden Ansprüche, des Weiteren umfassend ein Justierungsmittel zum Vorjustieren der Größe der vorbestimmten Dosis.

12. Injektionsvorrichtung nach Anspruch 11, wobei das Justierungsmittel eine eine justierbare Position definierende Geometrie umfasst, die dazu angepasst ist, eine Einschränkung einer relativen Bewegung zwischen dem ersten modusdefinierenden Element und dem zweiten modusdefinierenden Element während der Dosiseinstellung zu definieren.

13. Injektionsvorrichtung nach Anspruch 12, wobei das Justierungsmittel zumindest einen Teil des zweiten modusdefinierenden Elements bildet.

## Revendications

1. Un dispositif d'injection pour l'injection de doses prédéterminées d'un médicament liquide, le dispositif d'injection comprenant :
- un mécanisme d'ajustement de dose qui peut être actionné pour ajuster une dose prédéterminée,
- un mécanisme d'injection qui peut être actionné pour faire en sorte qu'une dose ajustée soit injectée par le dispositif d'injection,
- un ressort de torsion qui est relié au mécanisme d'ajustement de dose d'une manière telle que l'actionnement du mécanisme d'ajustement de dose provoque le stockage d'énergie dans le ressort de torsion, et le ressort de torsion étant relié au mécanisme d'injection d'une manière telle que l'actionnement du mécanisme d'injection fasse en sorte que l'énergie qui avait été précédemment stockée dans le ressort de torsion soit libérée, l'énergie libérée étant utilisée pour faire en sorte que la dose ajusté soit injectée,
- un premier élément de définition de mode et un second élément de définition de mode qui sont mobiles angulairement l'un par rapport à l'autre en réponse à l'actionnement du mécanisme de réglage de dose, et qui sont mobiles axialement l'un par rapport à l'autre entre une première position dans laquelle le mécanisme d'ajustement de dose peut être actionné pour ajuster une dose et une seconde position dans laquelle le mécanisme d'injection peut être actionné pour injecter une dose ajustée,
dans lequel les éléments de définition de mode sont conformés d'une manière telle que les mouvements axiaux mutuels entre la première position et la seconde position ne soient possibles que pour un nombre limité de positions angulaires mutuelles, lesdites positions angulaires correspondant chacune à l'ajustement d'une dose prédéterminée de médicament liquide.

2. Un dispositif d'injection selon la revendication 1, dans lequel les éléments de définition de mode sont conformés d'une manière telle que les mouvements axiaux mutuels entre la première position et la seconde position ne soient possibles que pour deux positions angulaires mutuelles, l'une desdites positions angulaires correspondant à l'ajustement d'une dose prédéterminée non nulle de médicament liquide et l'autre desdites positions angulaires correspondant à une dose nulle.

3. Un dispositif d'injection selon la revendication 1 ou 2, dans lequel le premier élément de définition de mode est pourvu d'une partie saillante, et le second élément de définition de mode est pourvu d'un nombre limité d'évidements angulairement distribués, chaque évidement étant conformé et dimensionné pour permettre à la partie saillante du premier élément de définition de mode de passer, permettant ainsi des mouvements axiaux mutuels du premier élément de définition de mode et du second élément de définition de mode seulement lorsque la position angulaire de la partie saillante correspond à la position angulaire de l'un desdits évidements.

4. Un dispositif d'injection selon l'une des revendications précédentes, dans lequel les éléments de définition de mode sont en outre aptes à être déplacés vers une troisième position axiale mutuelle, et dans lequel les mouvements angulaires mutuels entre les éléments de définition de mode sont empêchés lorsque les éléments de définition de mode sont positionnés dans la troisième position axiale.

5. Un dispositif d'injection selon la revendication 4, dans lequel des ensembles associés de dents sont formés sur le premier élément de définition de mode et le second élément de définition de mode, et dans lequel lesdits ensembles coopérants de dents viennent en prise lorsque les éléments de définition de mode sont dans la troisième position axiale.

6. Un dispositif d'injection selon la revendication 4 ou 5, dans lequel la troisième position axiale est disposée entre la première position axiale et la seconde position axiale.

7. Un dispositif d'injection selon l'une des revendications 4 à 6, dans lequel il n'est pas possible d'actionner le mécanisme d'ajustement de dose pour ajuster une dose lorsque les éléments de définition de mode sont dans la troisième position axiale.

8. Un dispositif d'injection selon l'une des revendications 4 à 7, dans lequel le mouvement des éléments de définition de mode de la troisième position axiale à la première position axiale n'est possible que lorsqu'une dose précédemment ajustée a été complètement injectée.

9. Un dispositif d'injection selon l'une des revendications précédentes, dans lequel les éléments de définition de mode sont disposés de manière à retourner automatiquement à la première position à l'achèvement d'une injection.

10. Un dispositif d'injection selon la revendication 9, dans lequel le retour automatique à la première position est obtenu grâce à des moyens de sollicitation mécaniques.

11. Un dispositif d'injection selon l'une des revendications précédentes, comprenant en outre des moyens de réglage pour prérégler la taille de la dose prédétermine.

12. Un dispositif d'injection selon la revendication 11, dans lequel les moyens de réglage comprennent une position réglable définissant une géométrie apte à définir une limite de mouvement angulaire relatif entre le premier élément de définition de mode et le second élément de définition de mode durant l'ajustement de la dose.

13. Un dispositif d'injection selon la revendication 12, dans lequel les moyens de réglage constituent au moins une partie du second élément de définition de mode.
